# EUROPEAN PATENT SPECIFICATION

(11) **EP 3 270 791 B1**
(45) Date of publication and mention of the grant of the patent: **01.01.2020**
(21) Application number: 16719503.1
(22) Date of filing: 16.03.2016
(51) Int. Cl.: A61B 10/02, A61B 90/00, A61B 10/00

(54) **DEVICE FOR THE SAMPLING OF THE EYE SURFACE BY IMPRINTING**
GERÄT ZUR GEWEBEGEWINNUNG DER AUGENOBERFLÄCHE DURCH AUFDRÜCKEN
DISPOSITIF POUR LA BIOPSIE DE LA SURFACE D'OEIL PAR IMPRESSION

(30) Priority: 16.03.2015 IT UB20150376
(43) Date of publication of application: 24.01.2018
(73) Proprietor: Fondazione G.B. Bietti per lo Studio e la Ricerca in Oftalmologia - ONLUS - IRCCS, 00198 Rome (IT); Università Campus Bio-Medico di Roma, 00128 Rome (RM) (IT)
(72) Inventor: MICERA, Alessandra, 00128 Rome (IT); ZOLLO, Loredana, 00128 Rome (IT); GHEZZI, Ilaria, 00128 Rome (IT); BALZAMINO, Bijorn Omar, 00198 Rome (IT); SGRULLETTA, Roberto, 00128 Roma (IT)
(74) Representative: Di Giovine, Paolo
(86) International application number: PCT/IB2016/051474
(87) International publication number: WO 2016/147122

(56) References cited:
- JP-B2- 3 074 544
- US-A1- 2006 084 077
- US-A1- 2011 319 789

## Description

### Technical field of the invention

The present invention relates to a device for the sampling of the eye surface, to the uses thereof in research and basic diagnostics in the veterinary, agro-feeding and medical field, in particular Ophthalmology. The object of the present invention is to provide a device for the sampling and analysis of the outer layers of the ocular surface, by impression cytology, solving the disadvantages of the known art.

### State of art

Impression cytology (or imprinting) on the ocular surface was used for the first time in 1954 by Larmande and Timsit for diagnosing a squamous neoplasm, but in Englang Egbert and his collaborators in 1977 used a cellulose membrane to analyse the presence of *goblet cells,* calyx-like cells secreting mucin, in patients with dry eye syndrome. These concluded then that the impression of the layer of surface cells could be obtained by pressing a cellulose membrane on the ocular surface for few seconds.

More recently Simon and Thiiel have described the use of a new membrane Biopore of nitrocellulose (0.22µm; Millicel sterilized Culture Plate Insert, Millipore Corp., Bedford, MA, USA) for the sampling and cytological analysis of layers of the ocular surface. The filter/membrane was positioned on the ocular surface, in the area of interest, and removed with a delicate peeling motion. After sampling, the membrane was removed/and cut quickly from the support and dipped in a sampling medium. This not invasive sampling technique was quickly adopted, improved and used for histological studies in patients affected by dry eye syndrome, Ocular Cicatricial Pemphigoid, limbal deficit, viral and bacterial infections, allergic forms, he, conjunctival and malignant melanoma and disorder caused by vitamin deficiency with alteration of the ocular surface. This sampling procedure, associated to a good sampling medium, keeps the morphology of the cells of the sampled conjunctival and/or corneal epithelium and it has the fundamental advantage of allowing an unlimited use of cytodiagnosis techniques, from the simplest basic colouring, to the immunohistochemistry/immunofluorescence (optical and confocal microscopy), to the extraction of DNA/RNA/proteins until coming to the extraction of the single cells to be analysed by means of cytofluorimetry. According to the needs dictated by the Ophthalmological survey, the material collected with impression cytology is commonly evaluated according to the morphological (distribution and density) and/or biomolecular parameters of the epithelial and muciparous cells, responsible for a healthy ocular surface. Other methods are based upon hydrolysis, glycolysis and hydroglycolysis to reduce the polymers of plastics in the starting monomers.

The impression cytology on ocular surface, furthermore, represents, an alternative to the highly invasive biopsy, by providing information by means of a minimally invasive approach and the absence of side effects or contraindications. The method result to be well tolerated by the patient.

The impression cytology can be performed in Ambulatory (routine), in presence (sampling of cornea and Limbus) or in absence of anaesthetic (conjunctival pick up), and the epithelium of the sampled area regenerates physically within 24 hours. In 24 hours it is not necessary to administer antibiotics. All this after approval, by the ethics committee, of the structure and/or written adhesion (informed consent signature) by the patient.

During the last years, the impression cytology has demonstrated to be a valid support in the differential diagnosis (bacterial/viral infections or limbal deficit) and above all during the last decade, the use thereof as research instrument (in Clinical Trials and base and translational research) has recorded a huge growth and it has considerably contributed to bring new information about some pathologies of the ocular surface (M.A THIEL, 1997).

The used membranes have different sizes and (rounded, square or half-moon) shapes, generally membranes of nitrocellulose both by Life Sciences (PALL) and by Millipore (Millicel) with 12/13 and 30mm and 0.2-0.4µm. These can be easily found and used for the microscopy with transmitted light and confocal microscopy techniques, being compatible with cellular colouring agents and/or chemical/fluorescent markers. The Millicel membranes further promote an excellent cellular growth, by offering exceptional opportunities for the cellular studies and they are available with Biopore (PTFE) membrane, MF-Millipore (esters mixed with cellulose) and polycarbonate; the first ones are suitable for immunofluorescence, observation of live cells and they have a low protein adsorption, whereas the second ones are suitable for exceptional anatomical and functional polarizations.

The circular plastic support whereon the Millipore membranes are positioned is a device for cellular cultures and therefore has four plastic supports which, in case of using for the impression cytology, are removed accurately with sterile pincers before the application on the ocular surface.

Differently from scraping and brushing, the conjunctival imprinting allows to obtain samples with a good cellular content, well preserved, not contaminated and it does not cause surface abrasion but only a gentle peeling. The samples, once preserved, can be evaluated afterwards in order to define the following cellular parameters: cellular arrangement/distribution, nucleous-cytoplasma ratio, presence of particular cellular categories (inflammatory and muciparous cells) and pathogens (bacteria, viruses, mycetes). It further allows to receive information about the ratios between the various epithelial (muciparous versus epithelial) elements and it allows to highlight intra-cytoplasmatic alterations (cellular inclusions in the chlamydiosis), loss of the intracellular adhesions and nuclear lesions (in the viral infections), by keeping unaltered the cell-cell bonds. On the Millipore membranes it is possible to perform not only techniques of optical microscopy (immunofluorescence or immunoperoxidase), but even biochemical and molecular analyses.

The impression cytology generally is performed during the outpatient visit, after written confirmation of the adhesion by the patient (informed consent) and explanation of the sampling and use destination procedure. Currently there are two sampling methods:
- A first method is based upon the positioning of the membrane disks, in particular of Biopore (PALL) type on the ocular surface for example by means of the help of tweezers with rounded edges. Such method results to be difficult to be implemented, if one is not skilled, as it is necessary to avoid touching the ocular surface with the tweezers. Furthermore, if not highly skilled, the operator risks to perform a wrong or partial (not sufficient) sampling by invalidating the pick-up itself.
- Another method is based upon the use of a Millipore sterile membrane, in a single packaging (Millicell-CM 0.4 micron PICM 012.550, Millipore Corp, Bedford, MA, USA). Such membrane is equipped with a cylindrical support in transparent plastic and it can be used without additional coatings. The initial use destination is for cell cultures (cell culture inserts), but currently it is used eve for sampling the ocular surface. In order to avoid ocular traumas, the three supports made of plastic existing on the cylinder edge have to be eliminated accurately with a sterile tweezers. The Millicell membrane is grasped directly by the hand of the operator and the membrane is delicately pressed onto the ocular surface for 3-5 seconds. The removal process is fast and safe and it provides to fix the sampled material before the repositioning in the original packaging for the transportation to the laboratory. There is no need to transfer the sample on micro-glasses or supports; it results to be a fast test, the sample is simple to be preserved. The cell adhesion to the membrane results to be quite stable, however during fixing it is possible to record loss of material as well as during the analysis procedures in laboratory.

However, currently the application of such membrane on the ocular epithelium still has a series of limits which make difficult the imprinting procedure even by skilled operators and it requires a high caution. The main limitations relate to:
- the need for exerting an adequate pressure onto the patient eye to allow on one side an effective imprinting but, on the other side, to avoid possible involuntary tears of the conjunctival tissue;
- the reduction of the operator's field of visibility due to the presence of his/her own hands on the patient's face;
- the possible discomfort sometimes complained by the patient and the consequent tendency to close the eyelids upon approaching the device;

- the need for locking the eyelids of the patient, with the consequent increase in discomfort of the same; this makes difficult the apposition of the membrane.
- the need, which often happens, to perform the sampling several times on the same area, by perturbing it more, as one has to repeat the pick-up in different days and by producing frequently small tears or traumas.

Currently on the market EyePrim (OPIA, France) is available, allowing to collect cells of the ocular surface by means of a syringe-shaped device which would ease both the pick-up and the sampling by the operator. The membrane, placed at the device end, has a half-moon geometry and it is constituted by a polyethersulfone filter, thicker than the Millipore membranes. Such device is able to perform a partial pick-up with respect to the one performed with the Millipore membranes due to the geometry itself of the used membranes; in fact, these have a half-moon shape differently from the circular geometry of the membranes illustrated in the previous pages, by reducing sensibly the pick-up area. The membrane is unlikely to be coloured and it results to be opaque to the optical route by preventing a clear acquisition of the image both in transmitted light and fluorescence. Such device is described in the American patent application US2011/319789. The state of art as above reconstructed then shows that the device for sampling the ocular surface by *imprinting* still have several disadvantages.

### Summary of the invention

The subject matter of the invention is described in the claims. The technical problem placed and solved by the present invention is then to provide a device for the ocular sampling, in particular for the sampling and analysis of the outer layers of the ocular surface by impression cytology, allowing to obviate the drawbacks mentioned above with reference to the known art.

Such problem is solved by a device according to claim 1. A kit for the sampling and analysis of the outer layers of the ocular surface by impression cytology, using such device, is also subject of the present invention.

Preferred features of the embodiments of the present invention are subject of the depending claims.

The device according to the present invention is able to improve significatively the current techniques for sampling the ocular surface, by intervening on a decrease in the time of applying the membrane, in particular of Millipore type, and discomfort for the patient, even if by increasing the effectiveness level of the procedure. In details, for planning such system the following requirements were defined:
- it has to allow the development of a method of simplified sampling for the operator, by improving the aspects and the previously mentioned currently found limitations,
- it has to allow the development of a method for sampling highly tolerated by the patient leading to an improvement in the level of safety, accuracy and repeatability of pick-up,
- it has to allow the development of a method for sampling facilitating the preservation of the sample storing system,
- it has to allow compensating the possible tremor and involuntary or inaccurate motions of the operator, through the presence of a support for the stable positioning of the device for the cytological pick-up,
- it has to allow reducing to the minimum the involuntary motions of the patient's eyelid, by facilitating the operator intervention and the procedure effectiveness, by means of using the device and not by means of the doctor's hands;
- it has to be ergonomic and to have an elementary geometry, which highlights the use simplicity thereof, but which at the same time is able to guide the operator in performing the procedure, thus extending the use possibility even to not skilled operators,
- it has to be able to integrate the nitrocellulose membrane, Millipore, already existing on the market and currently used for the impression cytology;
- it has to allow the sampling even in areas difficult to be reached in the maximum safety for the patient,
- it has to reduce the possibility of error by the operator by means of creating a standardized method and a guided procedure,
- it has to allow obtaining an extremely simple method, effective and which potentially can be used as instrument for the local alteration prediction,
- it has to allow a sampling procedure which preserves the morphology of the conjunctival and/or corneal epithelium and has the fundamental advantage of allowing an unlimited use of cytodiagnosis techniques in different medical, and not, fields;
- it has to have a weight lower than 300 grams and sizes compatible with those of the ocular orbit so as to ease the positioning thereof and to reduce the discomfort for the patient.

Therefore, a device was planned which in every aspect can ease the cytological picking-up method for the operator and which is further planned according to the directives existing in this respect.

Other advantages and features and use modes of the present invention will be evident from the following detailed description of some embodiments, shown by way of example and not for limitative purpose.

### Brief description of the figures

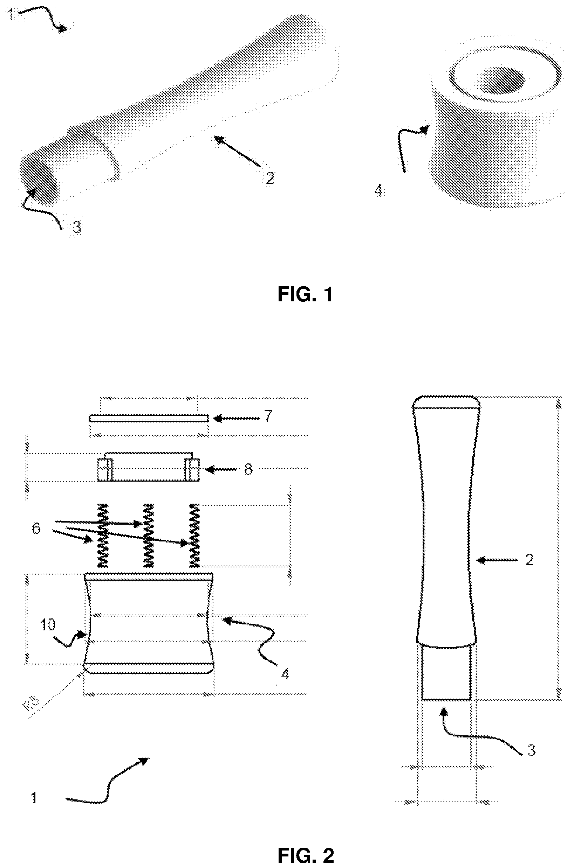

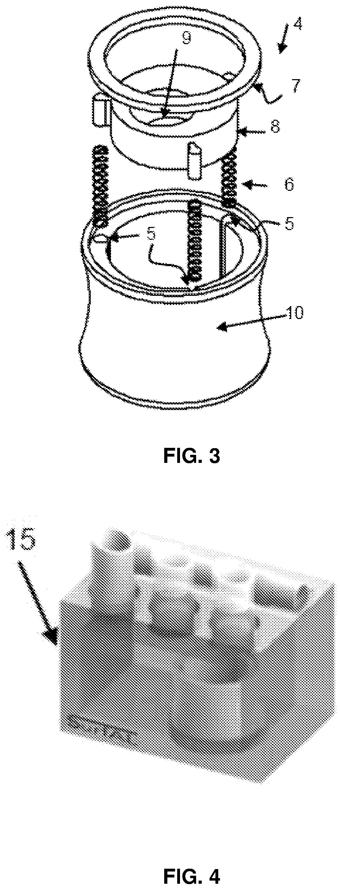

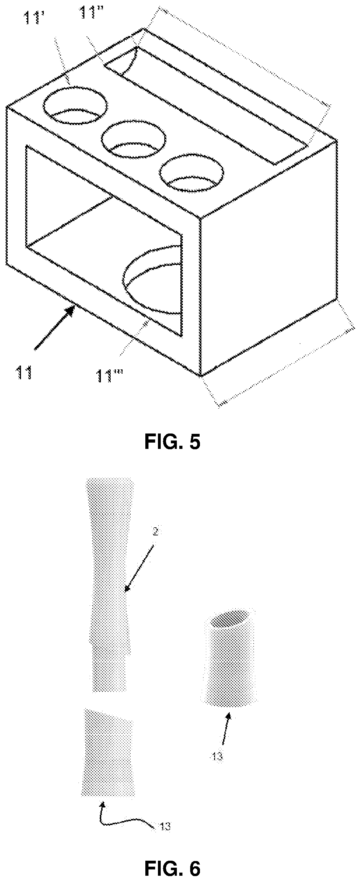

The figures of the enclosed drawings will be referred to, wherein:
▪ figure 1, is a perspective and general representation of the device according to a preferred embodiment of the present invention;
▪ figure 2 represents a side view of the device of figure 1;
▪ figure 3 is an exploded representation of the second element of the device of figures 1 and 2;
▪ figure 4 is a perspective and general representation of the kit according to a preferred embodiment of the present invention;
▪ figure 5 is a perspective representation of the supporting element of the kit of figure 4;
▪ figure 6 is a side view of the first element of the device of figure 1 and of the closing element of said first element.

### Detailed description of preferred embodiments

By firstly referring to figures 1 and 2, a device according to a preferred embodiment of the invention is designated as a whole with 1.

In the description sizes of the device are shown, not with limitative purposes, but established after the studies performed on the human eye morphology, so as to adapt advantageously to the eye without creating trouble.

The device comprises a first element 2 with substantially elongated shape having at one end a hollow portion 3 apt to house a membrane suitable for the ocular sampling.

The first element 2 (designated also as sub-system A or *pickup stick*) was devised for a double use mode: coupled with the second element 4 of the device (designated also as *periocular base*) for an assisted sampling, or in *stand-alone* mode for a manual sampling. The first element 2 advantageously will have an ergonomic shape, so as to be easy to be handled by the operator. The hollow portion 3 at one end thereof will be suitable to house a membrane for the ocular sampling, in particular it will be a circular cavity suitable to house the membrane of *Millipore* type.

The possibility of using said first element 2, even in *stand-alone* mode, allows performing an easy and safe sampling even in patients having pathologies therefor the eyeball has different morphological features than normal, it projects more or has smaller sizes, and which would prevent the correct positioning of the second element 4 of the device. The two elements (first and second element, respectively) are then suitable to be reversibly connected therebetween before and after use.

According to the embodiment represented in the figures the first element 2 has a cylindrical shape, in particular a solid cylindrical, slightly flared shape and at the lower end it has a hollow cylindrical section 3, with a circular base with an outer diameter of about 14 mm and an inner diameter of about 12 mm. This could be arranged for positioning the *Millipore* membrane with the support thereof, as already existing in the packaging itself.

The overall length of said first element is comprised between 80 and 90 mm, in particular 87 mm. The geometry and the sizes of said first element 2 were selected so that it could be handled easily by the operator during the sampling, even by increasing the distance of his/her hands from the patient's eye. An increase in the field of vision of the operator with respect to the membrane direct use follows and, consequently, a more controlled positioning of the membrane onto the ocular surface.

The second element 4, represented in figure 1 acts as support for the first element 2, which is inserted in properly sized housing and it can slide on guides 5 until reaching the ocular surface. Elastic means 6 comprising 3 compression springs inserted in the linear guides 5 allow a passive adjustment of the forces interacting with the tissues, by drastically increasing the interaction safety and reducing considerably the risk of damages. The joint use of the two elements of the device then allows an assisted sampling, with increase in the level of accuracy, repeatability and safety.

The device then allows a targeted and reproducible sampling; it is able to filter tremor or unskilled motions by the operator and to increase safety in the interaction with the ocular surface of the patient, by avoiding risks of damages to the tissues.

According to the embodiment represented in figures 1-5 the second element 4 has a hollow cylindrical geometry, preferably with a side curvature, so as to allow greater ergonomicity. Said second element 4 comprises a main body 10 preferably with an outer diameter of 42mm and an inner diameter of 30mm so as to obtain a complete winding of the orbit and not to obscure the vision of the patient. The outer diameter preferably will have slightly larger sizes than those of the orbit to allow a stable positioning around the eye, to limit the motion of the eyelids and to not create discomfort or constriction in the patient. Said main body will have a base with curve profile, with radius equal to about 2mm, in order to allow a more comfortable, safer and less troublesome rest onto the patient's face, the height of said main body preferably will be about 30 mm.

Said second element 4, in the main body thereof 10 has guiding means 5, in particular three linear guides arranged at 120° one with respect to the other one for housing a mobile disc 8 sliding along the hollow cylinder of the main body of the element. The elastic means 6 is positioned in the linear guides, in particular three compression springs having the following features: inner diameter equal to 2.55 mm, outer diameter equal to 3.05 mm, 9 active turns, free length (if not subjected to load) equal to 20.57 mm, length at compaction equal to 7.75mm. The three springs have a stiffness, K, equal to 0.16 N/mm and they result to resist to a maximum force equal to 2.14 N, measured experimentally during *imprinting* procedures performed in laboratory. The presence of the mobile disc 8 on linear guides allows guiding the operator in performing the cytological pick-up, by compensating possible tremor or involuntary motions. The use of the properly sized springs 6 guarantees the control of the maximum forces applicable by the operator's hand during imprinting, thus increasing safety in the interaction with the ocular surface and avoiding tears of tissues. The material of the springs is stainless steel with a working temperature between -200° C and +280°C.

The mobile element 8 shaped like a disc has a hollow portion, designated in figures with numeral 9, advantageously tilted by about 15 degrees coherently with the natural curvature of the human eye. Said hollow portion 9 (or hole) will be suitable for inserting the first element 2 by allowing the sampling in different points of the ocular surface by simply rotating the second element 4. The discharge of the mobile disc from the second element will be advantageously avoided by the presence of a circular element 7 fastened to the upper end of the periocular support. This for example could have the sizes of 2 mm in thickness, 41 mm of outer diameter and 38 mm of inner diameter and be fastened, for example by interlocking in the upper portion of said second element.

The second element thus was planned for allowing the insertion of the first element in the hollow portion 9 tilted by 15 degrees. In fact the inner disc facilitates the sampling of the involved portion by addressing the operator's hand in pre-established areas for the sampling, by avoiding possible errors or inaccuracies by the operator. In this way the analysis of the side areas results to be possible without modifying the membrane and without the risk of damaging the ocular wall.

According to an embodiment the complete device could have a weight not exceeding 250 grams excluding the springs. The used material, excluding the springs, for creating the device could be for example polycarbonate or PVC, both materials guarantee the possibility of sterilizing in autoclave (as Tg=150 °C), or by means of gamma rays.

A subject of the present invention is also a kit for the ocular sampling, in particular for the sampling and analysis of the outer layers of the ocular surface by impression cytology.

By making reference to figure 4, this shows a preferred embodiment of the kit designated as a whole with 15. The kit could include one or more devices according to the present invention and/or one or more membranes for the ocular sampling, preferably of the *Millipore* type.

Said kit could further comprise even one or more closing elements 13 of said first element. The closing elements, designated in figure 6 with numeral 13, are suitable to preserve the sample by avoiding the deterioration thereof. These closing elements have a specular geometry with respect to the first element so as to allow the perfect housing of the lower portion of the first element inside thereof. The upper portion thereof preferably is tilted by 15 degrees, in order to allow a perfect coupling with the first element, and it has a portion including a liquid quantity for preserving the membrane after sampling, for example up to a maximum of 300µl. The kit then could further comprise even solutions suitable for the biochemical, confocal or molecular analysis of the collected samples.

The kit could further include a supporting structure, such as for example that represented in figure 5 and designated as a whole with the numeral 11, comprising one or more seats 11" for said first element, one or more seats 11''' for said second element and one or more seats 11' for said closing elements. Detailed description of the method for using the device and the kit according to the preferred embodiment of the present invention represented in figures 1-6:
Both for the manual sampling (with *stand-alone* use of the first element 2) and for the assisted sampling (with joint use of the first and second element 4), the procedure for assembling the device to the membrane used for the sampling results to be the same.

At first the plastic support of the membrane have to be removed, by using sterile tweezers, by making attention to remove each residue in order to obtain a smooth surface which does not run the risk of scratching the ocular epithelium.

The passages are schematized here below:
- insertion of the membrane, comprising the plastic support thereof, in the lower portion of the first element.
- insertion of the first element in the hollow portion 9 (hole) of the second element
- detection of the area of interest and widening of the eyelid area of the patient;
- positioning of the second element on the ocular orbit with orientation of the hole according to the area of interest;
- by means of grasping the second element 4 with the thumb and middle finger, applying a slight pressure onto the upper end of the first element 2 with forefinger, until reaching the ocular surface;
- keeping the pressure for 4-5 seconds and then removing the device from the eye;
- positioning the device on a flat surface and extracting the first element from the second element;
- inserting the first element into one of the closing elements 13 supplied with the kit 15, based upon the analyses to be performed;
- possible preparation of the documents accompanying the shipment/delivery to the laboratory which will perform the analysis, in line with the existing rules relating shipment of biological sample for research/diagnosis.

The device of the present invention results easy to be used, effective and extremely interesting as instrument for predicting the alteration of the ocular surface. The possibility of performing a biochemical analysis, in addition to the molecular one, would allow to identify protein *markers* which, with respect to the molecular markers, would allow to overcome post-transcriptional or post-translational locks and/or modifications. At the same time, a quick and safe outpatient *screening* would allow a prompt pharmacological response and a quicker therapeutic application, an essential requirement in the Ophthalmological practice. The flexibility of this device allows the the quick transfer of the impression cytology to the ophthalmological sanitary service and subsequently to the other, sanitary and not (for example veterinary).
- The use of the device in the basic research and translational field allows a quick and standardized sampling, independently from the operator's experience, by offering a real support for *clinical trials* and national and International multicentre studies. All this assisted by a quick shipment to the reference centres for the analyses or analyses on site.
- The use in sanitary field (outpatient, primary care physician, first aid) would allow: a timely base diagnosis of predictive type and/or therapeutic monitoring; a support to the clinic by evaluating *markers* validated for diagnosis, prognosis and/or therapeutic monitoring (differential analysis in case of bacterial and/or viral infections or over-infections as well as evaluation of the effectiveness of a therapy or pharmacological damage, through cytotoxicity tests).

Examples of application areas of the device and of the kit according to the present invention are:
- medical field (Outpatient: Ophthalmology; Dermatology; Dentology);
- veterinary field (Outpatient for analyses of infections);
- agro-feeding field (*onsite* sampling for analyses of adulterations).

The present invention has been sofar described by referring to some preferred embodiments. It is to be meant that other embodiments belonging to the same inventive core may exist, as defined by the protective scope of the herebelow reported claims.

### Comparative experiments

PAS staining on impression cytologies performed with EYEPRIM™ device and comparison with sampling method with Millicell membrane (Millipore) currently used in the most part of the basic and translational research studies.

The comparison between the two devices was performed by evaluating the pre-Analytical (during sampling) and Analytical (laboratory survey) attitudes.

### 1. Pre-Analytical: comparison by pick-up type

In case of EYEPRIM™ (device described in US2011/319789) the pick-up results to be partial and it seems not so easy to be implemented as the device is not "ergonomic" and therefore it does not replace in a satisfying way the classic method with Millicell support (Millipore membrane). The reluctance would lie in the fact that the syringe-like device results to be uncomfortable at the time of the ocular sampling. To this purpose, the device according to the present invention brings an improvement both in the classical outpatient sampling (Millicell) and compared to the EYEPRIM™ method. This results from the analysis of questionnaires developed specifically for the device analysis. In the specific case, the device according to the present invention was analysed for physical and mechanical features as sub-system 1 (A), sub-system 2 (B) and sub-system 1 assisted by the sub-system 2 (A+B). The voluntary subject (operator) expressed his/her opinion relating the used methods and his/her sensations, having also tested EYEPRIM™.
- 85% of consulted operators consider the sub-system A light, ergonomic and easy to be handled, they have no comments relating the shape and features and they do not show interest in using it.
- 60% of consulted operators, instead, show little interest in the sub-system B by considering it bulky or with not ideal geometry for the use thereof; however, they recognize a potential improvement in the sampling procedure and a higher effectiveness and simplicity. To this purpose the device was made lighter and refined in the shapes.

On the whole, about 70% of operators consider to be satisfied by the proposed device and evaluate it simple to be used, by giving a good/optimum overall judgment and they prefer it to EYEPRIM™.

Ultimately, from this study it resulted that the device according to the present invention sub-system 1 is more ergonomic, lighter and more flexible than the EYEPRIM™ device. The device resulted to be easy to be used as, since it is not in the "syringe-like" type, the operator has not to impart a pressure which, above all in the least skilled people, could destabilize the hand at the moment of impact onto the ocular surface. The spring has no buffer systems. Such limitation is compensated in the device sub-system 1 assisted by the sub-system 2 which allows not only a targeted sampling but even a calibration of the membrane-ocular surface contact force (protection effect).

Furthermore the device has a supported environmental impact as "if one wishes it" only the membrane can be replaced. The device comes not directly in contact with the patient and, if wished, the specialist can bring it always with him/her for the "in office" procedures. The cap, by offering the fastening possibility at time of the pick-up, has a "convenience" in the shipment to the analysis laboratory.

### 2. Analytical: comparison of the membranes by means of histological coloration according to PAS method and acquisition in transmitted light digital microscopy.

The samplings reported hereinafter were performed by an Ophthalmologist specialist on volunteers (four samplings on three healthy volunteers, in outpatient seat) and the process was performed in laboratory by a specialized operator (Biologist). The membranes were removed from the support (technique of detachment by means of surgical knife) and subjected to PAS coloration (Periodic Acid Schiff Hotchkiss - MCManus #04-130802, Bioptica), a differential coloration which is performed directly onto the prefixed membrane (citofix; BioOptica). The coloration passages were: 1. hydration in distilled water (3min); 2. Treatment with 1 % Periodic Acid (5min) and washing in distilled water (1min); 3. Coloration with Schiff base reagent (2min) and washing in distilled water (3min); 4. nuclear countercoloration with Mayer Hematoxylin (2min) and differentiation in spring water (5min); 5. balancing in phosphate buffer and positioning of the coloured membrane on cover glass for closing with cover glass (mountant based upon glycerol/gelatin in phosphate buffer).

The membrane of the device EYEPRIM™ results to be opaque to the passage of the light beam (direct microscope), by preventing a clear acquisition of the transmitted light image. On the contrary, the device according to the present invention, as it does not change the type of perfectly tested membrane, allows a clear and distinct acquisition of the images by means of optical microscopy and in some cases immunofluorescence.

## Claims

1. A device (1) for ocular sampling, in particular for the sampling and analysis of the outer layers of the ocular surface by impression cytology, comprising:
- a first element (2) with substantially elongated shape having at one end a first hollow portion (3) apt to house a membrane suitable for the ocular sampling;
- a second element (4), comprising guiding means (5) for said first element (2) apt to allow sliding of said first element relative to said second element , and elastic means (6) positioned in such a way as to exert an opposing force against said first element (2) during said sliding, wherein said hollow portion (3) is a circular portion and the membrane is in particular a membrane of millipore type, wherein said second element (4) has a main body (10) with substantially tubular shape open at both ends, said main body comprising said guiding means (5) and said elastic means (6) **characterised in that** said second element (4) further comprises a movable element (8) apt to slide along said main body (10) by means of said guiding means (5) and wherein said elastic means (6) comprise a plurality of springs (6) inserted in said guiding means (5);
wherein said movable element (8) has a second hollow portion (9) for inserting said first element (2).

2. The device (1) according to claim 1 further comprising a locking element (7) of said movable element (8), in particular wherein said locking element has a circular shape and it is fixed on said second element (4).

3. The device (1) according to claim 1 wherein the second hollow portion (9) of said movable element (8) is tilted by about 15 degrees.

4. The device (1) according to anyone of the previous claims wherein the first hollow portion (3) of said first element (2) has a circular cross section with a tilting of about 15 degrees.

5. The device (1) according to anyone of the previous claims wherein said elastic means (6) exerts an opposing force against said first element (2) of maximum value equal to 2.14 N.

6. The device (1) according to anyone of the previous claims wherein said first (2) and second (4) element are suitable to be reversibly connected therebetween before and after use.

7. The device (1) according to anyone of the previous claims further comprising a membrane of millipore type, wherein said membrane of millipore type is housed on said circular portion (3).

8. A kit (15) comprising the device according to any one of the previous claim further comprising:
- one or more closing elements (13) of said lower portion (3) of said first element, and/or
- one or more solutions suitable for the biochemical, confocal or molecular analysis of said ocular samples, and/or
- a supporting structure (11) comprising one or more seats (11 ") for said first element (3) and/or one or more seats (11''') for said second element and/or one or more seats (11') for said closing elements (13) and/or
- one or more membranes suitable for the ocular sampling.

## Patentansprüche

1. Vorrichtung (1) zur okularen Probeentnahme, insbesondere zur Probeentnahme von und zur Analyse der äußeren Schichten der Augenoberfläche durch Impressionszytologie, umfassend:
ein erstes Element (2) mit im Wesentlichen länglicher Form, das an einem ersten Ende einen ersten hohlen Abschnitt (3) aufweist, welcher geeignet ist, eine Membran zu beherbergen, die für die okulare Probeentnahme geeignet ist;
ein zweites Element (4), das Führungsmittel (5) für das erste Element (2) umfasst, die geeignet sind, ein Gleiten des ersten Elementes relativ zu dem zweiten Element zu ermöglichen, und elastische Mittel (6), die auf solche Weise angeordnet sind, um eine Gegenkraft gegen das erste Element (2) während des Gleitens auszuüben, wobei der hohle Abschnitt (3) ein kreisförmiger Abschnitt ist und die Membran insbesondere eine Membran des Millipore-Typs ist, wobei das zweite Element (4) einen Hauptkörper (10) mit im Wesentlichen rohrförmiger Gestalt aufweist, der an beiden Enden offen ist, wobei der Hauptkörper die Führungsmittel (5) und die elastischen Mittel (6) umfasst, **dadurch gekennzeichnet, dass** das zweite Element (4) weiterhin ein bewegbares Element (8) umfasst, das geeignet ist, entlang des Hauptkörpers (10) mittels der Führungsmittel (5) zu gleiten, und wobei die elastischen Mittel (6) eine Vielzahl von Federn (6) umfassen, die in die Führungsmittel (5) eingefügt sind;
wobei das bewegbare Element (8) einen zweiten hohlen Abschnitt (9) zum Einfügen des ersten Elements (2) umfasst.

2. Vorrichtung (1) nach Anspruch 1, die weiterhin ein Verriegelungselement (7) des bewegbaren Elements (8) umfasst, wobei insbesondere das Verriegelungselement eine Kreisform aufweist und an dem zweiten Element (4) befestigt ist.

3. Vorrichtung (1) nach Anspruch 1, wobei der zweite hohle Abschnitt (9) des bewegbaren Elements (8) um etwa 15 Grad geneigt ist.

4. Vorrichtung (1) nach einem der vorhergehenden Ansprüche, wobei der erste hohle Abschnitt (3) des ersten Elements (2) einen kreisförmigen Querschnitt mit einer Neigung von etwa 15 Grad aufweist.

5. Vorrichtung (1) nach einem der vorhergehenden Ansprüche, wobei die elastischen Mittel (6) eine Gegenkraft gegen das erste Element (2) mit einem Maximalwert von 2,14 N ausüben.

6. Vorrichtung (1) nach einem der vorhergehenden Ansprüche, wobei das erste (2) und das zweite (4) Element geeignet sind, vor und nach Verwendung reversibel miteinander verbunden zu sein.

7. Vorrichtung (1) nach einem der vorhergehenden Ansprüche, die weiterhin eine Membran des Millipore-Typs umfasst, wobei die Membran des Millipore-Typs in dem kreisförmigen Abschnitt (3) beherbergt ist.

8. Kit (15) mit der Vorrichtung nach einem der vorhergehenden Ansprüche, weiterhin umfassend:
- ein oder mehrere Verschlusselemente (13) des unteren Abschnitts (3) des ersten Elements, und/oder
- ein oder mehrere Lösungen, die für die biochemische, konfokale, oder molekulare Analyse der okularen Proben geeignet sind, und/oder
- eine Tragestruktur (11), die ein oder mehrere Sitze (11 ") für das erste Element (3) und/oder einen oder mehrere Sitze (11''') für das zweite Element und/oder ein oder mehrere Sitze (11') für die Verschlusselemente (13) umfasst, und/oder
- ein oder mehrere Membranen, die für die okulare Probeentnahme geeignet sind.

## Revendications

1. Dispositif (1) d'échantillonnage oculaire, en particulier pour l'échantillonnage et l'analyse des couches extérieures de la surface oculaire par cytologie par impression, comprenant :
- un premier élément (2) de forme sensiblement allongée ayant, à une première extrémité, une première partie creuse (3) apte à loger une membrane convenant pour l'échantillonnage oculaire ;
- un deuxième élément (4) comprenant un moyen de guidage (5) pour ledit premier élément (2) apte à permettre un coulissement dudit premier élément par rapport audit deuxième élément, et un moyen élastique (6) positionné de façon à exercer une force opposée contre ledit premier élément (2) durant ledit coulissement,
dans lequel ladite partie creuse (3) est une partie circulaire et la membrane est en particulier une membrane de type millipore, dans lequel ledit deuxième élément (4) a un corps principal (10) de forme sensiblement tubulaire ouvert aux deux extrémités, ledit corps principal comprenant ledit moyen de guidage (5) et ledit moyen élastique (6), **caractérisé en ce que** ledit deuxième élément (4) comprend en outre un élément mobile (8) apte à coulisser le long dudit corps principal (10) au moyen dudit moyen de guidage (5), et dans lequel ledit moyen élastique (6) comprend une pluralité de ressorts (6) insérés dans ledit moyen de guidage (5) ;
dans lequel ledit élément mobile (8) a une deuxième partie creuse (9) pour l'insertion dudit premier élément (2).

2. Dispositif (1) selon la revendication 1, comprenant en outre un élément de verrouillage (7) dudit élément mobile (8), en particulier dans lequel ledit élément de verrouillage a une forme circulaire et est fixé sur ledit deuxième élément (4).

3. Dispositif (1) selon la revendication 1, dans lequel la deuxième partie creuse (9) dudit élément mobile (8) est inclinée d'environ 15 degrés.

4. Dispositif (1) selon l'une quelconque des revendications précédentes, dans lequel la première partie creuse (3) dudit premier élément (2) a une section transversale circulaire avec une inclinaison d'environ 15 degrés.

5. Dispositif (1) selon l'une quelconque des revendications précédentes, dans lequel ledit moyen élastique (6) exerce une force opposée contre ledit premier élément (2) ayant une valeur maximale égale à 2,14 N.

6. Dispositif (1) selon l'une quelconque des revendications précédentes, dans lequel lesdits premier (2) et deuxième (4) éléments conviennent pour être connectés entre eux de manière réversible avant et après utilisation.

7. Dispositif (1) selon l'une quelconque des revendications précédentes, comprenant en outre une membrane de type millipore, dans lequel ladite membrane de type millipore est disposée sur ladite partie circulaire (3).

8. Kit (8) comprenant le dispositif selon l'une quelconque des revendications précédentes, comprenant en outre :
- un ou plusieurs éléments de fermeture (13) de ladite partie inférieure (3) dudit premier élément, et/ou
- une ou plusieurs solutions convenant pour l'analyse biochimique, confocale ou moléculaire desdits échantillons oculaire, et/ou
- une structure de support (11) comprenant un ou plusieurs sièges (11 ") pour ledit premier élément (3) et/ou un ou plusieurs sièges (11''') pour ledit deuxième élément et/ou un ou plusieurs sièges (11') pour lesdits éléments de fermeture (13), et/ou
- une ou plusieurs membranes convenant pour l'échantillonnage oculaire.
